# EUROPEAN PATENT SPECIFICATION

(11) **EP 4 228 507 B1**
(45) Date of publication and mention of the grant of the patent: **08.07.2026**
(21) Application number: 21801192.2
(22) Date of filing: 14.10.2021
(51) Int. Cl.: A61B 5/145, G01N 27/416, G01N 27/36, A61B 5/1495, A61B 5/00

(54) **TESTING APPARATUS**
TESTVORRICHTUNG
APPAREIL DE TEST

(30) Priority: 14.10.2020 GB 202016324
(43) Date of publication of application: 23.08.2023
(73) Proprietor: Softcell Medical Limited, Aberdeen AB10 1UT (GB)
(72) Inventor: MCINTOSH, Kirsty, Aberdeen Aberdeenshire AB12 5FA (GB); MENNA, Francesco, 80121 Napoli (IT)
(74) Representative: Murgitroyd & Company
(86) International application number: PCT/GB2021/052659
(87) International publication number: WO 2022/079436

(56) References cited:
- US-A- 2 256 733
- US-A- 3 855 095
- US-A- 4 608 148
- US-B2- 8 262 878

## Description

### TECHNICAL FIELD

The present invention relates to testing apparatus in the form of a pH probe apparatus. More particularly, the present invention relates to such apparatus for use in the assessment of the health or condition of soft tissues, the composition of body fluids, and with modification of the probe insertion technique, the assessment of the health or condition of bone.

### BACKGROUND

A variety of devices for testing and monitoring pH of tissue and fluids are generally known. As these have been found by the applicant to suffer from accuracy of reading and stability of reading issues, the applicant devised an improved pH sensor, which is disclosed WO2013186513. This relates to a pH sensor adapted to be inserted into soft tissues, such as muscle, fat or other organs e.g. heart, lung, kidney, liver, pancreas, bowel, skin, brain, and within the layers of the gastrointestinal wall, or body fluids such as, blood, cerebrospinal fluid, urine, peritoneal fluid, joint fluid, vitreous humor, and gastrointestinal contents.

Whilst the apparatus of the applicant's earlier application has represented an advancement over prior devices, they have developed aspects of the apparatus, its method of manufacture and use, with the aim of yet further improvement.

US2256733 discloses a device for the electrical determination of pH, comprising a glass electrode having a hydrogen ion responsive membrane and a glass tubular extension.

From US3855095 it is known to provide a glass electrode assembly including inner and outer glass tubes in which the internal half-cell of the electrode is sealed by a glass seal to the inner tube close to the ion sensitive bulb by means of wave energy, rather than a gas flame, so as to avoid contamination of electrolyte in the electrode.

US4608148 relates to a combination pH/reference electrode having two electrochemical references being thermally insulated from the sensing membrane and being thermally adjacent each other.

US8262878 discloses a controlled activation sensor assembly for monitoring pH or other constituents, involving a means of combining and configuring specific hydrophilic and dielectric materials in such a way as to allow an antimony/reference electrode pH sensor to be packaged and stored dry yet become fully hydrated to an activated state after exposure to aqueous liquids.

According to an aspect of the present invention, there is provided a sensor for determining pH of soft tissues, bodily fluids or bone of an organism, the sensor comprising: a sensor body having a sensor tip formed at one end, the sensor body including a spherical glass bulb and a cylindrical glass capillary tube extending from the glass bulb, wherein the sensor body is formed to include an inner cavity containing a reference buffer solution; an insulating structure formed around and within the capillary tube, wherein the insulating structure further includes: one or more concentric inner tubes, the inner tubes being concentric when more than one inner tube is provided; wherein the one or more inner tubes extend fully within the inside of the glass capillary tube up to the glass bulb and beyond the end point of the glass capillary tube, the inside of the one or more inner tubes and the bulb thereby forming the inner cavity containing the reference buffer solution; a sensor wire disposed within the capillary tube having a first end extending into a central portion of the glass bulb, and a second end extending beyond the capillary tube; a middle tube covering the capillary tube and the one or more inner tubes, including an end point of the capillary tube that overlaps the one or more inner tubes; a probe main tube formed over and extending past the middle tube; the probe main tube covering the sensor wire; and an outer bushing (9) covering the probe main tube.

Preferably the insulating structure is formed from different materials.

Optionally, the insulating structure is selected from plastics materials.

Preferably, the insulating structure is selected from one or more of: epoxy materials, LDPE, LLDPE co-extrudable adhesive resins, or block copolymers.

Optionally, the insulating structure is provided with a coupling material, in which case optionally wherein the coupling material is provided with one or more air pockets.

Preferably, a pair of inner tubes is provided, and wherein the pair of inner tubes are formed from GRILAMID (RTM).

Optionally, the middle tube is formed from GRILAMID (RTM).

Preferably, the probe main tubing is formed from PEBAX ^{™}.

Optionally, the outer bushing is formed from PEBAX ^{™}.

Preferably, the glass bulb is formed from heating pH sensitive glass to between 800-1000 degrees C.

Optionally, the impedance of the glass bulb is less than 2 Gohms.

Preferably, the glass bulb is aged for at least 3 months.

Optionally, the ratio of the impedance of the glass bulb and the insulated capillary tube is in the range 1:5 to 1:9.

According to a further aspect of the present invention, there is provided a system for use in testing pH of soft tissues, bodily fluids or bone, the system having one or more sensors as set out above and monitor apparatus, the monitor apparatus interacting with each of the one or more probe apparatuses to provide real time data of probe apparatus operational parameters and soft tissue, bodily fluids or bone well-being or disease state or severity of injury.

Preferably, the monitor apparatus has a plurality of connections for respective probe apparatuses.

Optionally, the monitor apparatus periodically interrogates each probe apparatus coupled thereto.

Particular advantages of the subject matter described in this application include probe apparatus that seeks to offer enhanced and consistent accuracy and stability of readings. The probe apparatus comes in a form that is sterile and ready for use, eliminating the need for calibration while also permitting a drift check to be performed prior to use to avoid clinically relevant errors and the ability to undertake a minor adjustment of drift prior to use if necessary.

From the monitor perspective, the apparatus provides a variety of alarms that currently do not exist with the pH monitoring systems of others. As it has multiple channels, the monitor can, for example, be used simultaneously to monitor multiple points on the patient. The period of use and function of each probe is independent of other probes.

The system also has the advantage of being pre-calibrated in the factory. Calibration immediately prior to use is time consuming and not without risk of error, such as mixing up and/or contaminating buffer solutions that could result in erroneous calibration and have undesirable clinical results.

The various aspects of the present invention can be practiced alone or in combination with one or more of the other aspects, as will be appreciated by those skilled in the relevant arts. The various aspects of the invention can optionally be provided in combination with one or more of the optional features of the other aspects of the invention. Also, optional features described in relation to one aspect can typically be combined alone or together with other features in different aspects of the invention. Any subject matter described in this specification can be combined with any other subject matter in the specification to form a novel combination.

Various aspects of the invention will now be described in detail with reference to the accompanying figures. Still other aspects, features, and advantages of the present invention are readily apparent from the entire description thereof, including the figures, which illustrates a number of exemplary aspects and implementations. The invention is also capable of other and different examples and aspects, and its several details can be modified in various respects, all without departing from the scope of the present invention, which is defined by the appended claims.

Accordingly, each example herein should be understood to have broad application, and is meant to illustrate one possible way of carrying out the invention, without intending to suggest that the scope of this disclosure, including the claims, is limited to that example. Furthermore, the terminology and phraseology used herein is solely used for descriptive purposes and should not be construed as limiting in scope. In particular, unless otherwise stated, dimensions and numerical values included herein are presented as examples illustrating one possible aspect of the claimed subject matter, without limiting the disclosure to the particular dimensions or values recited. All numerical values in this disclosure are understood as being modified by "about". All singular forms of elements, or any other components described herein are understood to include plural forms thereof and vice versa. Language such as "including", "comprising", "having", "containing", or "involving" and variations thereof, is intended to be broad and encompass the subject matter listed thereafter, and is not intended to exclude other additives, components, integers or steps. Likewise, the term "comprising" is considered synonymous with the terms "including" or "containing" for applicable legal purposes. Thus, throughout the specification and claims unless the context requires otherwise, the word "comprise" or variations thereof such as "comprises" or "comprising" will be understood to imply the inclusion of a stated integer or group of integers but not the exclusion of any other integer or group of integers.

Any discussion of documents, acts, materials, devices, articles and the like is included in the specification solely for the purpose of providing a context for the present invention. It is not suggested or represented that any or all of these matters formed part of the prior art base or were common general knowledge in the field relevant to the present invention.

In this disclosure, whenever a composition, an element or a group of elements is preceded with the transitional phrase "comprising", it is understood that we also contemplate the same composition, element or group of elements with transitional phrases "consisting essentially of", "consisting", "selected from the group of consisting of', "including", or "is" preceding the recitation of the composition, element or group of elements and vice versa. In this disclosure, the words "typically" or "optionally" are to be understood as being intended to indicate optional or non-essential features of the invention which are present in certain examples but which can be omitted in others without departing from the scope of the invention.

References to directional and positional descriptions such as upper and lower and directions e.g. "up", "down" etc. are to be interpreted by a skilled reader in the context of the examples described to refer to the orientation of features shown in the drawings, and are not to be interpreted as limiting the invention to the literal interpretation of the term, but instead should be as understood by the skilled addressee.

Embodiments of the present invention will now be described by way of example and with reference to the accompanying drawings, of which:-
Figure 1 shows a longitudinal cross-section through the tip of a probe in accordance with the present invention;
Figure 2 shows an axial cross-sectional view through the probe of Figure 1 at section X-X; and
Figure 3 shows a view of a monitor connected to a probe in accordance with the present invention.

### Probe Structure and Method of Manufacture

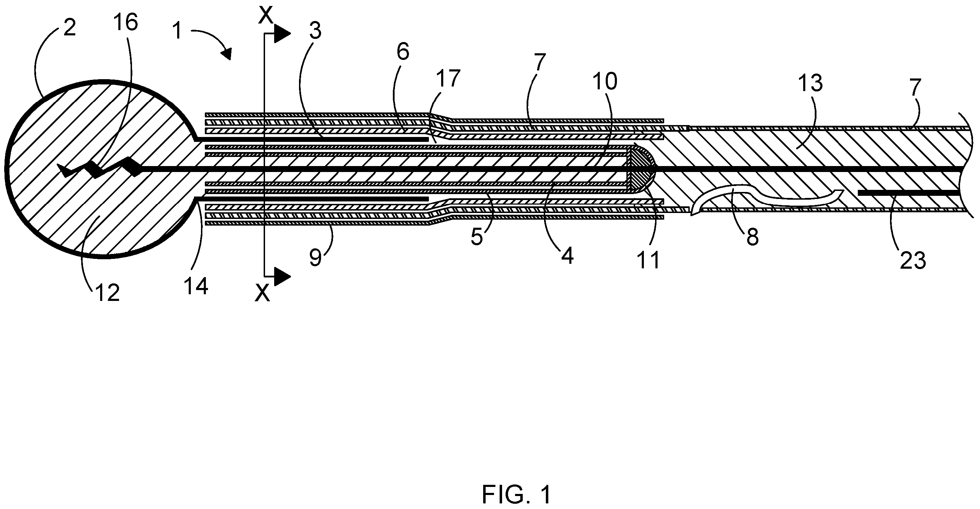

As shown in Figure 1, a pH sensor probe 1 generally comprises a glass bulb 2 connected to a glass capillary tube 3. The glass bulb provides a sensing end component and the capillary tube provides a sleeve for housing a sensor signal wire 10, that terminates at one end at a sensor electrode 16. Whilst glass is discussed in relation to this embodiment other suitable materials, where appropriate may be employed.

The glass bulb 2 has a diameter in the range of 1.6 to 1.8mm. In the preferred implementation, the glass bulb 2 may have a maximum diameter of substantially 1.8mm. In this connection, the glass bulb is manufactured to high tolerances, including overall spherical shape, constant wall thickness, thinness of wall, symmetry and limited connection to glass capillary tube to maintain glass bulb pH sensitive properties and to improve the insulation of the glass bulb. As will be described in greater detail below, as part of the microfabrication process, the glass bulb 2 is connected to the glass capillary tube 3 forming a junction 14. The dimensions of the pH sensor 1 , the glass bulb 2 and the glass capillary tube 3 may be appropriately reduced and/or adjusted to suit requirements, for example for paediatrics or neonate applications or for large/small animals.

Preferably, the wall thickness of the glass bulb 2 is substantially 0.15mm to 0.5mm, and more preferably 0.2mm. It will be appreciated that alternative applications of the pH sensor probe 1 may require a different preferred wall thickness for the glass bulb. The diameter of the glass capillary tube 3 is preferably 1.8 mm, and the preferred wall thickness is in the range 0.08 to 0.12mm. Preferably, the wall thickness of the glass capillary tube is substantially 0.1 mm. In this regard, in general the outer diameter of the capillary tube should be the same as or smaller than the diameter of the glass bulb.

In this connection, the glass bulb diameter is similar or preferably slightly smaller than the outermost diameter of the capillary tube, including any outer sleeves or bushings. In this way, the glass bulb is protected during insertion and removal.

In this connection, the glass bulb 2 is formed by high precision blowing after heating to extremely high temperatures, namely in the range 800-1000 degrees C. In this manner, the glass is relatively liquid, namely with a preferred viscosity to allow the desired wall thickness to be achieved with minimal material being used to create each bulb. In this regard, the insufflation of the glass bulb is preferably undertaken using compressed air at 0.5 ATM, such that this part of the manufacturing method involves a highly delicate low pressure process.

The thinner the glass of the glass bulb 2, the more responsive it is to pH change i.e. the speed of response when placed in different pH environments. Further, the more consistent the glass bulb shape is, the more consistent the wall thickness is which also improves the speed of response since a larger surface area of the bulb is reacting to changes in surrounding pH.

The type of glass used for the bulb is pH sensitive glass manufactured by CLR and consequently highly sensitive to hydrogen ions. The connection of the glass bulb 2 to the glass capillary tube 3 at junction 14 is symmetrical and limited to maintain the pH sensitive properties of the glass bulb 2 and to improve the insulation of the glass bulb.

The glass bulb 2 is formed using both techniques and glass material that result in a structure having a very low impedance relative to the stem of the glass capillary tube 3, which is formed from a glass material, such as quartz glass, having a relatively high impedance.

Preferably, the impedance of the glass bulb is less than 2 Gohms whilst the impedance of the glass capillary tube is over 10 Gohms. In some implementations, the impedance of the glass bulb is preferably in the range of 1.0. to 2.5 Gohms.

In a preferred implementation, the ratio of the impedance of the glass bulb 2 to the glass capillary tube 3 is in the range 1 :5 to 1 :9.

The glass capillary tube is preferably formed from quartz glass, which is very resistant to temperature. Quartz glass starts to become soft between 1200 - 1600°C. At 800 -1000°C, the flame temperature for heating the glass bulb is high enough to reduce the pH sensitive glass to a very molten state but not enough to melt the glass capillary tube. As such, there is a clean/sharp transition between the two types of glass where they meet. This sharp demarcation helps to maintain the insulation of the glass bulb.

During the manufacturing process, the connection or junction 14 between the glass bulb 2 and the glass capillary tube 3 is inspected at various stages to ensure even welding. Such inspections may take place after the bulb has been blown, after assembly of the sensor unit, and after final probe assembly has been completed.

The glass bulb 2 is furthermore aged to enhance stability of the probe. The aging process begins when the glass bulb 2 is first attached to the glass capillary tube 3. The aging process can take up to 3 months or more and is preferably a minimum of 3 months with no effective maximum. The size of the pores in the glass become more consistent in size with ageing, whether this is natural or accelerated in some way. In this respect, the glass will function as a semi- permeable membrane.

The aging may be natural or can be accelerated using, for example, chemical methods. This may entail using 5% Fluoric Acid (Hydroxyfluoric acid).

The end result is consistency of size of the pores in the glass of the glass bulb 2, enhancing the probe reading stability, and optimising responsiveness to changes in pH and accuracy of readings as the whole surface area of the glass bulb 2 is responsive to changes in environmental pH.

Impedance testing of the combined glass bulb and capillary tube structure is used to confirm that the insulation of the sensor unit has been maintained during the aging process so that the impedance of the glass bulb remains below 2 Gohms, whereby the impedance of the glass bulb is relatively low compared with that of the capillary tube.

As discussed above the glass bulb 2 is attached to a capillary glass tube 3 at junction 14. In a further manufacturing step, two internal tubes 4, 5 are formed and dimensioned to be inserted within the glass capillary tube 3. In this connection, and as shown in Figure 1 , the two inner or internal tubes 4 and 5 act to form the remainder of the capillary component. The internal tubes 4, 5 insulate the sensor probe up to the point of connection 14 of the capillary glass tube 3 with the glass bulb 2. They also extend beyond the end point 17 of the capillary glass tube 3 to ensure a signal wire 10 does not make contact with the wall of the capillary glass tube 3.

The internal tubes 4, 5 are located so that they both extend inside the capillary glass tube 3. Preferably, the internal tubes 4, 5 extend fully within the inside of the glass capillary tube 3 up to the junction 14 at which the glass bulb 2 is connected to the glass capillary tube 3.

The internal tubes 4, 5 are preferably both made from GRILAMID (RTM), a polyamide. In this connection, GRILAMID (RTM) benefits from high flexibility and insulating properties. Alternative suitable materials may however include polymers/plastics with similar properties as well as other materials including metals, ceramics, and carbon materials. Whilst providing a single internal tube is an option, a pair of such concentric internal tubes 4, 5 are provided in this embodiment to enhance the insulating effect.

The internal tubes 4, 5 as such provide an internal insulating component to the glass capillary tube 3. In this respect, the internal insulating component affords shielding, e.g. to RF interference, and strength to the relatively delicate glass capillary tube 3. The internal insulating component moreover prevents direct contact of the signal wire with the capillary tube.

A coupling material such as an adhesive, for example an epoxy, is preferably provided between the internal layers within the glass capillary tube 3, such as tubes 4, 5 to improve the overall insulation and to key the components together. During manufacture, the adhesive is preferably under-loaded within the glass capillary tube 3, e.g. at the facing surfaces of the internal tubes 4, 5. In this way, air pockets are naturally formed in the adhesive, such air pockets being preferred as they enhance the overall insulating effect. The internal tubes are, in this regard, not applied directly to the capillary tube but are coupled thereto with the coupling material.

In this regard, whilst as described above, a coupling material may be provided between the internal layers 4, 5 of the glass capillary tube, such a coupling material may equally be provided between any facing surfaces of the capillary component or the insulating component. Further, whilst described above air pockets can be formed within the adhesive between the internal tubes 4, 5, it will be understood that one or more air/gas pockets or bubbles may be provided within the coupling material, wherever it is provided to thereby enhance the insulating effect.

Suitable coupling materials may include epoxies, acrylates, cyanoacrylate, silicones and urethanes. One such material may be cyanoacrylate adhesive.

In further preferred implementations, an epoxy material is used.

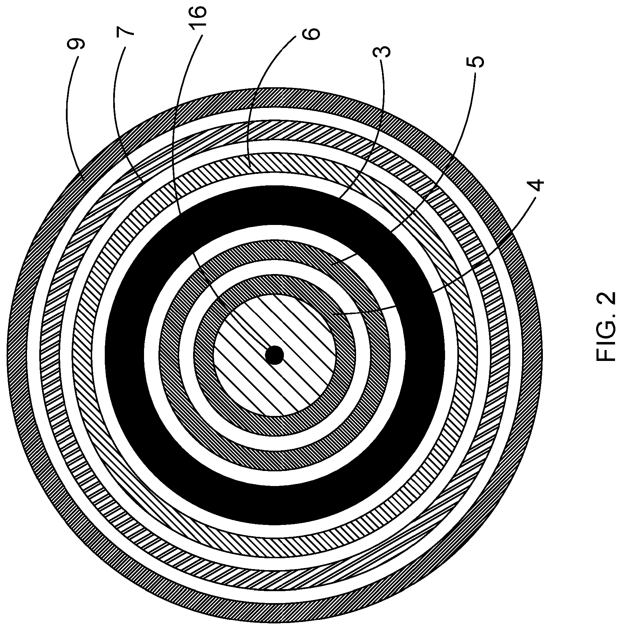

As shown in Figure 2, during manufacturing signal wire 10 in the form of a silver wire 10, is inserted into the internal tubes 4, 5 until the tip of the silver wire 10 terminates at the sensor electrode 16 in the centre of the spherical glass bulb 2. The electrode 16 may in this respect simply comprise a portion of the wire 10 folded over upon itself, or can comprise a dedicated element having a configuration for presenting an increased surface area for enhanced sensitivity.

The internal cavity of the glass bulb 2, glass capillary tube 3, and internal tubes 4, 5 are then filled with a technical buffer solution 12 of known pH. In the preferred implementation, the buffer solution 12 has a pH of 7.0.

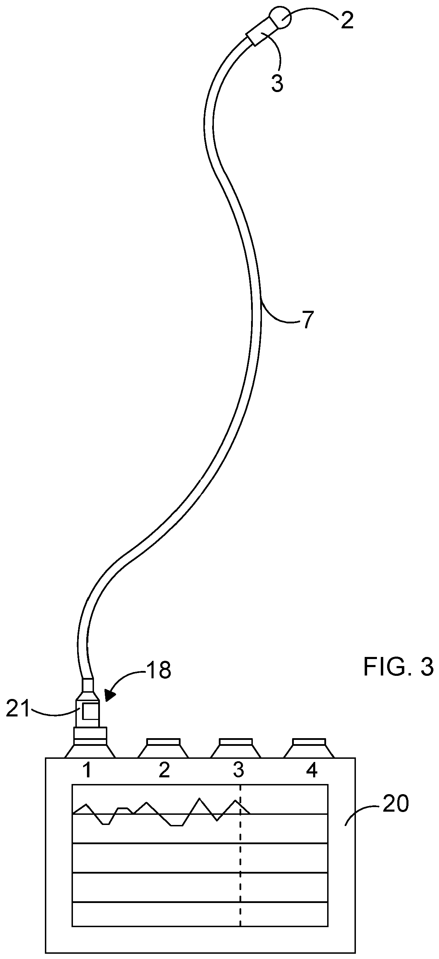

The internal tubes 4, 5 are preferably sealed at one end with a plug 11 , for example of epoxy, that provides a complete seal around the silver wire 10 to prevent any escape of buffer solution 12. The remaining portion of the silver wire 10 extends into the probe main tubing 7 where it is welded/soldered onto an insulated silver wire that extends throughout the length of the probe tubing and terminates at a suitable electrical connector 18 suitable for connection into a monitor 20 shown in Figure 3.

In this connection, whilst the internal tubes 4, 5 are shown extending out from the glass capillary tube 3, the ends of the internal tubes may be closer than shown to the end 17 of the glass capillary tube, so that the plug 11 can be used to seal not only the internal tubes 4, 5 but also the glass capillary tube 3. Again as shown in Figure 1, a middle tube 6 covers all of the sensor probe 1 from the connection of the glass bulb 2 and the capillary tube 3, signal wire 10 and the two internal insulating tubes 4, 5. The middle tube 6 also provides an additional seal between the glass capillary tube 3 and the internal tubes 4, 5, and increases the overall strength and rigidity of the sensor probe 1. The middle tube 6 also provides a high strength bonding surface for securely adhering the probe main tubing 7 to the sensor probe 1 thereby further preventing the sensor probe 1 from separating from the probe main tubing 7.

Whilst alternative suitable materials may be used, the middle tube 6 is preferably made from GRILAMID, a polyamide that is a lightweight polymer elastomer.

Probe main tubing 7 is preferably also made from PEBAX (RTM). In a preferred example, the probe main tubing 7 may extend for 1.60 to 1.65m from the connection 14 of the glass bulb 2 and capillary tube 3 and encompasses the capillary tube 3, the tubes 4, 5 the middle tube 6. The probe main tubing 7 and thus fully sheathed and insulated wire 10 extends to a monitor 20 shown in Figure 3. The probe main tubing may be longer or shorter depending upon clinical requirements.

The sensor probe 1 also includes outer bushing 9 that is formed and/or moulded around the outer surface of the probe main tubing 7. The outer bushing 9 is preferably substantially 1cm long and supports the join of the probe main tubing 7 onto the sensor probe 1. Whilst the outer bushing 9 is preferably made from PEBAX, other suitable biocompatible materials may be used.

Cyanoacrylate glue (resin) is preferably used to seal the sensor and is preferably also provided between the two inner insulating tubes 4 and 5 to improve the overall insulation. The glue is also provided between the outer of the two inner tubes and the inner aspect of the glass capillary tube. In this respect, coupling material such as adhesive may be provided between one or more of the layers from outer bushing 9 to the internal tube 4. Adhesive may in this respect be provided between all the layers from the outer bushing 9 to the internal tube. The adhesive may be an epoxy or suitable alternative.

The coupling material is preferably present between all layers from innermost to outermost. It is preferably inserted using capillary action to draw the glue in between the tubular materials.

A sterile cotton thread 8 or alternative suitable component exits the plastic probe tubing through a small puncture immediately adjacent to the proximal part of the outer bushing 9. A technical 7.00 pH buffer solution 12 is provided within the glass bulb. This may for example be, 51302047 [Mettler Toledo] Buffer Solution, pH 7.00],

The cotton thread 8 is soaked in Friscolyt 13 from within the probe sheath and from the Friscolyt in which the probe tip is stored up until use. The cotton thread 8 acts as an electrical conducting salt bridge. A reference electrode 23 is provided in the probe main tubing 7.

As discussed above, and as shown in Figures 1 and 2, the sensor probe 1 has insulation formed of several layers, preferably of different plastics, some or all of which are themselves preferably separated by layers of adhesive resin/fluid filled space, e.g. air pockets or bubbles or liquids such as gel applied in an "onionskin" manner. The insulating effect is so effective that after completing the insulating process, the only detectable reduced area of impedance remains the glass bulb 2. A further advantage of the internal insulating tubes and the coupling material arrangement is that the reference pH 7.0 buffer that is within the glass sensor bulb does not make contact with the capillary glass, so no theoretical chemical interactions or electrical connections are possible.

In this regard, an internal insulation component comprises insulation provided internally of the glass capillary tube 3, such as internal tubes 4, 5 and any adhesive materials, air pockets or other materials there-between, whilst an external insulation component comprises those components externally of the glass capillary tube 3, such as the middle tube 6, the probe main tube 7 and the outer bushing 9, as well as any adhesive materials, air pockets or other materials provided there-between.

The internal and external insulation components, as well as the glass capillary tube and any adhesive materials, air pockets or other materials provided therebetween contribute to insulating the sensor wire from the environment external to the probe tip.

By inserting an insulation layer inside the capillary tube, another level of insulation is created, namely between wiring attached to the glass sensor, the buffer solution and the inner aspect of the capillary glass tube, thereby maximising the insulation between the sensor and the capillary glass tube since the capillary glass tube has essentially been surrounded or isolated by the inner and outer insulating layers of the insulation component that are sealed with epoxy glue.

As mentioned above, the sensor probe 1 comprises a memory storage device 21 , for example a memory PCB, provided in the connector 18, on which is stored details of a full calibration carried out on the sensor probe at manufacture. The full calibration at manufacture includes calibrating the sensor probe using at least two buffer solutions in order to plot a calibration curve or gradient for the probe apparatus. In this regard, at point of use, the calibration details can be readily accessed and checked. If the calibration curve has for example drifted, a suitable adjustment can be made or if the drift is outside of tolerances the sensor probe 1 may be designated improper for use.

In this regard, impedance checks may be carried out at manufacture in the factory for confirming the insulation is functioning correctly or after the probe has been fully assembled and immediately prior to use Figure 3 shows the system of the present invention involving a pH sensor probe 1 as described above connected to a monitor 20 of the present invention. The monitor 20 is configured to interact with one or more dedicated pH sensor probes to thereby create a single or multiple channel pH monitoring system.

The interactions between the pH sensor probe 1 and monitor 20 may include (in the following non exhaustive list)

Transfer of pre-loaded (in the factory) probe specific data to monitor including individualised probe calibration, unique probe number, and date of manufacture and pre-set shelf life.

The pre-set calibration data permits the monitor to set its parameters to match the unique properties of the probe.

Probe performance/quality/safety check with immobilisation of probe if performance is below pre-set acceptable standards.

Drift adjustment (zeroing) if required.

Probe expiry of shelf life feature with immediate immobilisation of probe.

Probe attempted reuse feature with immediate immobilisation of probe.

Probe dislodgement alert - visual and audible.

Probe exceeding period of use alert - visual and audible.

Overall clinically relevant pH level alerts - visual and audible.

Compensation of pH Probe performance based on a drift check.

### Monitor specific alerts:

Low battery alert.

Detection and rejection of a pH Probe that has already been used - single use pH Probes.

Detection and rejection of a pH Probe whose characteristics have failed. Dislodgement alert.

Active disablement.

The monitor can preferably accommodate and record data from up to 4 probes simultaneously. Probes can be connected and disconnected singly without interfering with the operation of other probes attached to the monitor.

The monitor graphical display may use a "traffic light" system of colours to highlight any pH levels that are of concern to the clinician. This colour scheme is used for the display of absolute pH numbers according to each probe in situ and a trend display of pH over time for each probe.

### Testing

Each pH sensor probe 1 and/or probe component is preferably subjected to impedance testing prior to assembly, during assembly and post assembly to optimise its performance characteristics and to ensure the quality of the probe insulation is maintained.

In this regard, prior to fully assembling each probe, the impedance of each glass bulb 2 (connected to the capillary tube) is tested to ensure that the impedance is < 2Gohms.

Prior to assembly, the impedance of each of the raw components of the sensor probe 1 are tested. Impedance testing is also performed during and after the assembly of the pH sensor probe 1 is completed. Probes that do not meet the impedance requirements are discarded. In this way, a comparison can be made between the impedance of the glass bulb 2 attached to the glass capillary tube 3 (stem) and the final pH sensor probe 1 .

Following full assembly of the pH sensor probe 1 , pH performance testing is repeated at intervals until stability of the readings has been achieved. Performance stability coincides with stability of the glass bulb 2 that is achieved when the glass bulb has been aged optimally. Following full assembly of the pH sensor probe 1 , checks can be undertaken to ensure that the impedance remains within the desirable range indicating that the insulation of the pH sensor probe 1 continues to function optimally. A satisfactory impedance combined with an adequate period of ageing of the glass bulb 2 used in manufacturing the pH sensor probe 1 will indicate that overall probe performance should be maintained. After manufacture, ageing of the glass sensor results in stabilisation of pH readings resulting in negligible reading drift. By combining our knowledge of glass sensor performance due to ageing and confirming that the glass sensor is still adequately insulated, by checking impedance, it will be possible, in some circumstances, to remove the need to undertake a pH drift check altogether. Therefore, immediately prior to use, in some circumstances, it is possible to use impedance (in isolation) of the sensor housed within the fully assembled probe as a functional performance check of the probe.

### Applications of the Probe

The pH sensor probe 1 and system of the present invention may be used to monitor the relative 'health' or "state" of any living tissue (human and other animals) in-vivo or ex-vivo or indeed dying or dead tissue that is sufficiently big enough to permit the probe to be inserted provided, it does not cause irreparable harm.

The pH sensor probe 1 and system can provide real time information on numerous acute medical issues/diseases as well as the effects of trauma.

The pH sensor probe 1 can also permit clinicians to observe the effects of different treatments, for example to monitor the progression of a virus such as COVID-19 or the treatment thereof. It is anticipated that the pH sensor probe 1 and monitoring system will ultimately be used to assess a wide variety of organ specific and general body conditions. In a further application, the pH probe apparatus and system may be used on dead tissue/organs/bodily fluids, for example as a forensic tool for working out the time of death. Although a few implementations have been described in detail above, other modifications are possible. For example, the precise number and details of the insulating layers may varying depending upon the requirements.

In addition, while various manufacturing steps have been described, it will be understood that it is not necessarily required to perform these manufacturing steps in this exact order or sequence to arrive at the same invention or achieve desirable results. Accordingly, other implementations are within the scope of the following claims.

## Claims

1. A sensor (1) for determining pH of soft tissues, bodily fluids or bone of an organism, the sensor comprising:
a sensor body having a sensor tip formed at one end, the sensor body including a spherical glass bulb (2) and a cylindrical glass capillary tube (3) extending from the glass bulb, wherein the sensor body is formed to include an inner cavity containing a reference buffer solution (12);
an insulating structure formed around and within the capillary tube, wherein the insulating structure further includes:
one or more concentric inner tubes (4, 5), the inner tubes being concentric when more than one inner tube is provided; wherein the one or more inner tubes extend fully within the inside of the glass capillary tube up to the glass bulb and beyond the end point of the glass capillary tube, the inside of the one or more inner tubes and the bulb thereby forming the inner cavity containing the reference buffer solution;
a sensor wire (10) disposed within the capillary tube having a first end extending into a central portion of the glass bulb, and a second end extending beyond the capillary tube;
a middle tube (6) covering the capillary tube and the one or more inner tubes, including an end point of the capillary tube that overlaps the one or more inner tubes;
a probe main tube (7) formed over and extending past the middle tube; the probe main tube covering the sensor wire; and an outer bushing (9) covering the probe main tube.

2. The sensor of claim 1, and any one or more of:
a) wherein the insulating structure is formed from different materials; or
b) wherein the insulating structure is selected from plastics materials; or
c) wherein the insulating structure is selected from one or more of: epoxy materials, LDPE, LLDPE co-extrudable adhesive resins, or block copolymers; or
d) wherein the insulating structure is provided with a coupling material, in which case optionally wherein the coupling material is provided with one or more air pockets.

3. The sensor of claim 1 or 2, and any one or more of:
a) wherein a pair of inner tubes (4, 5) is provided, and wherein the pair of inner tubes are formed from GRILAMID (RTM); or
b) wherein the middle tube (6) is formed from GRILAMID (RTM); or
c) wherein the probe main tubing (7) is formed from PEBAX ^{™}; or
di) wherein the outer bushing (9) is formed from PEBAX ^{™}.

4. The sensor of any preceding claim, wherein the glass bulb (2) is formed from heating pH sensitive glass to between 800-1000 degrees C, in which case optionally any one or more of:
a) wherein the impedance of the glass bulb is less than 2 Gohms; or
b) wherein the glass bulb is aged for at least 3 months.

5. The sensor of any preceding claim, wherein the ratio of the impedance of the glass bulb (2) and the insulated capillary tube (3) is in the range 1:5 to 1:9.

6. A system for use in testing pH of soft tissues, bodily fluids or bone, the system having one or more sensors of any preceding claim and monitor apparatus (20), the monitor apparatus interacting with each of the one or more probe apparatuses to provide real time data of probe apparatus operational parameters and soft tissue, bodily fluids or bone well-being or disease state or severity of injury.

7. The system of claim 6, wherein the monitor apparatus (20) has a plurality of connections (18) for respective probe apparatuses, in which case optionally wherein the monitor apparatus periodically interrogates each probe apparatus coupled thereto.

## Patentansprüche

1. Ein Sensor (1) zum Bestimmen des pH-Werts von Weichgeweben, Körperflüssigkeiten oder Knochen eines Organismus, wobei der Sensor Folgendes beinhaltet:
einen Sensorkörper mit einer an einem Ende gebildeten Sensorspitze, wobei der Sensorkörper einen kugelförmigen Glaskolben (2) und ein zylindrisches Glaskapillarrohr (3), das sich von dem Glaskolben erstreckt, umfasst, wobei der Sensorkörper so geformt ist, dass er einen Innenhohlraum umfasst, der eine Referenzpufferlösung (12) enthält;
eine Isolierstruktur, die um das und innerhalb des Kapillarrohrs gebildet ist, wobei die Isolierstruktur ferner Folgendes umfasst:
ein oder mehrere konzentrische Innenrohre (4, 5), wobei die Innenrohre konzentrisch sind, wenn mehr als ein Innenrohr vorgesehen ist; wobei sich das eine oder die mehreren Innenrohre vollständig innerhalb des Inneren des Glaskapillarrohrs bis zu dem Glaskolben und über den Endpunkt des Glaskapillarrohrs hinaus erstrecken, wobei das Innere des einen oder der mehreren Innenrohre und der Kolben dadurch den Innenhohlraum bilden, der die Referenzpufferlösung enthält;
einen Sensordraht (10), der innerhalb des Kapillarrohrs angeordnet ist und ein erstes Ende, das sich in einen zentralen Abschnitt des Glaskolbens erstreckt, und ein zweites Ende, das sich über das Kapillarrohr hinaus erstreckt, aufweist;
ein Mittelrohr (6), das das Kapillarrohr und das eine oder die mehreren Innenrohre abdeckt und einen Endpunkt des Kapillarrohrs umfasst, der das eine oder die mehreren Innenrohre überlappt;
ein Sondenhauptrohr (7), das über das Mittelrohr gebildet ist und sich über dieses hinaus erstreckt; wobei das Sondenhauptrohr den Sensordraht abdeckt; und eine äußere Hülse (9), die das Sondenhauptrohr abdeckt.

2. Sensor gemäß Anspruch 1, und eines oder mehrere von Folgenden:
a) wobei die Isolierstruktur aus unterschiedlichen Materialien gebildet ist; oder
b) wobei die Isolierstruktur aus Kunststoffmaterialien ausgewählt ist; oder
c) wobei die Isolierstruktur aus einem oder mehreren von Folgenden ausgewählt ist: Epoxidmaterialien, LDPE, koextrudierbaren LLDPE-Klebstoffharzen oder Blockcopolymeren; oder
d) wobei die Isolierstruktur mit einem Kopplungsmaterial versehen ist, wobei in diesem Fall optional das Kopplungsmaterial mit einem oder mehreren Lufteinschlüssen versehen ist.

3. Sensor gemäß Anspruch 1 oder 2, und eines oder mehrere von Folgenden:
a) wobei ein Paar Innenrohre (4, 5) vorgesehen ist und wobei das Paar Innenrohre aus GRILAMID (RTM) gebildet ist; oder
b) wobei das Mittelrohr (6) aus GRILAMID (RTM) gebildet ist; oder
c) wobei der Sondenhauptschlauch (7) aus PEBAX ^{™} gebildet ist; oder
di) wobei die äußere Hülse (9) aus PEBAX ^{™} gebildet ist.

4. Sensor gemäß einem der vorhergehenden Ansprüche, wobei der Glaskolben (2) durch Erhitzen von pH-empfindlichem Glas auf zwischen 800-1000 Grad C gebildet ist, wobei in diesem Fall optional eines oder mehrere von Folgenden gilt:
a) wobei die Impedanz des Glaskolbens weniger als 2 Gohm beträgt; oder
b) wobei der Glaskolben mindestens 3 Monate lang gealtert ist.

5. Sensor gemäß einem der vorhergehenden Ansprüche, wobei das Verhältnis der Impedanz des Glaskolbens (2) und des isolierten Kapillarrohrs (3) in dem Bereich von 1 : 5 bis 1 : 9 liegt.

6. Ein System zur Verwendung bei der Prüfung des pH-Werts von Weichgeweben, Körperflüssigkeiten oder Knochen, wobei das System einen oder mehrere Sensoren gemäß einem der vorhergehenden Ansprüche und eine Überwachungsvorrichtung (20) aufweist, wobei die Überwachungsvorrichtung mit jeder der einen oder der mehreren Sondenvorrichtungen zusammenwirkt, um Echtzeitdaten zu Betriebsparametern der Sondenvorrichtungen sowie zum Gesundheitszustand oder Krankheitszustand von Weichgewebe, Körperflüssigkeiten oder Knochen oder zum Schweregrad einer Verletzung bereitzustellen.

7. System gemäß Anspruch 6, wobei die Überwachungsvorrichtung (20) eine Vielzahl von Anschlüssen (18) für jeweilige Sondenvorrichtungen aufweist, wobei in diesem Fall optional die Überwachungsvorrichtung jede daran gekoppelte Sondenvorrichtung periodisch abfragt.

## Revendications

1. Un capteur (1) destiné à la détermination du pH de tissus mous, de fluides corporels ou d'os d'un organisme, le capteur comprenant :
un corps de capteur présentant une pointe de capteur formée au niveau d'une extrémité, le corps de capteur incluant une ampoule en verre sphérique (2) et un tube capillaire en verre cylindrique (3) s'étendant à partir de l'ampoule en verre, le corps de capteur étant formé de façon à inclure une cavité interne contenant une solution tampon de référence (12) ;
une structure isolante formée autour et au sein du tube capillaire, la structure isolante incluant en outre :
un ou plusieurs tubes internes concentriques (4, 5), les tubes internes étant concentriques lorsque plus d'un tube interne est fourni ; les un ou plusieurs tubes internes s'étendant entièrement au sein de l'intérieur du tube capillaire en verre jusqu'à l'ampoule en verre et au-delà du point d'extrémité du tube capillaire en verre, l'intérieur des un ou plusieurs tubes internes et de l'ampoule formant ainsi la cavité interne contenant la solution tampon de référence ;
un fil de capteur (10) disposé au sein du tube capillaire présentant une première extrémité s'étendant jusque dans une portion centrale de l'ampoule en verre, et une deuxième extrémité s'étendant au-delà du tube capillaire ;
un tube médian (6) recouvrant le tube capillaire et les un ou plusieurs tubes internes,
incluant un point d'extrémité du tube capillaire qui chevauche les un ou plusieurs tubes internes ;
un tube principal de sonde (7) formé par-dessus et s'étendant plus loin que le tube médian ; le tube principal de sonde recouvrant le fil de capteur ; et une douille externe (9) recouvrant le tube principal de sonde.

2. Le capteur de la revendication 1, et un quelconque ou plusieurs parmi :
a) dans lequel la structure isolante est formée à partir de matériaux différents ; ou
b) dans lequel la structure isolante est sélectionnée parmi des matériaux plastiques ; ou
c) dans lequel la structure isolante est sélectionnée parmi un ou plusieurs parmi : des matériaux époxy, du LDPE, des résines adhésives co-extrudables de LLDPE, ou des copolymères séquencés ; ou
d) dans lequel la structure isolante est pourvue d'un matériau de couplage, auquel cas facultativement dans lequel le matériau de couplage est pourvu d'une ou plusieurs poches d'air.

3. Le capteur de la revendication 1 ou de la revendication 2, et un quelconque ou plusieurs parmi :
a) dans lequel une paire de tubes internes (4, 5) est fournie, et dans lequel la paire de tubes internes est formée à partir de GRILAMID (RTM) ; ou
b) dans lequel le tube médian (6) est formé à partir de GRILAMID (RTM) ; ou
c) dans lequel le tube principal de sonde (7) est formé à partir de PEBAX ^{™} ; ou
di) dans lequel la douille externe (9) est formée à partir de PEBAX ^{™}.

4. Le capteur de n'importe quelle revendication précédente, dans lequel l'ampoule en verre (2) est formée par chauffage de verre sensible au pH jusqu'à entre 800 et 1000 degrés C, auquel cas facultativement un quelconque ou plusieurs parmi :
a) dans lequel l'impédance de l'ampoule en verre est inférieure à 2 Gohms ; ou
b) dans lequel l'ampoule en verre est vieillie pendant au moins 3 mois.

5. Le capteur de n'importe quelle revendication précédente, dans lequel le rapport de l'impédance de l'ampoule en verre (2) et du tube capillaire isolé (3) est compris dans la plage de 1/5 à 1/9.

6. Un système destiné à être utilisé afin de tester le pH de tissus mous, de fluides corporels ou d'os, le système présentant un ou plusieurs capteurs de n'importe quelle revendication précédente et un appareil de surveillance (20), l'appareil de surveillance interagissant avec chacun des un ou plusieurs appareils de sonde de façon à fournir des données en temps réel sur les paramètres opérationnels d'appareil de sonde et sur l'état de santé, l'état de maladie ou la gravité d'une blessure des tissus mous, des fluides corporels ou des os.

7. Le système de la revendication 6, dans lequel l'appareil de surveillance (20) présente une pluralité de connexions (18) destinées à des appareils de sonde respectifs, auquel cas facultativement dans lequel l'appareil de surveillance interroge périodiquement chaque appareil de sonde couplé à celui-ci.
